(19) Europäisches Patentamt
European Patent Office
Office européen des brevets

(11) **EP 3 786 963 B1**

(12) **EUROPEAN PATENT SPECIFICATION**

(45) Date of publication and mention
of the grant of the patent:
**12.04.2023 Bulletin 2023/15**

(21) Application number: **20192211.9**

(22) Date of filing: **21.08.2020**

(51) International Patent Classification (IPC):
**G16C 60/00** *(2019.01)*

(52) Cooperative Patent Classification (CPC):
**G16C 60/00;** G16C 20/30; G16C 20/50

(54) **DESIGN PROGRAM AND DESIGN METHOD**

ENTWURFSPROGRAMM UND ENTWURFSVERFAHREN

PROGRAMME ET PROCÉDÉ DE CONCEPTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.08.2019 JP 2019154807**

(43) Date of publication of application:
**03.03.2021 Bulletin 2021/09**

(73) Proprietor: **FUJITSU LIMITED
Kanagawa 211-8588 (JP)**

(72) Inventors:
• **ANAZAWA, Toshihisa**
**138542 Singapore (SG)**
• **IMANAKA, Yoshihiko**
**Kawasaki-shi, Kanagawa 211-8588 (JP)**
• **SHIMOKAWA, Satoshi**
**Kawasaki-shi, Kanagawa 211-8588 (JP)**
• **OSHIMA, Hirotaka**
**Kawasaki-shi, Kanagawa 211-8588 (JP)**

(74) Representative: **Haseltine Lake Kempner LLP
Cheapside House
138 Cheapside
London EC2V 6BJ (GB)**

(56) References cited:
CN-A- 110 021 371     US-A1- 2012 180 453
US-A1- 2015 060 743     US-B2- 9 656 878

**Description**

FIELD

The embodiment relates to a design program for a composition of a perovskite type crystal structure and a design method.

BACKGROUND

A material having a perovskite type crystal structure exhibits various physical properties such as ferroelectricity depending on a composition. Therefore, a material having a perovskite type crystal structure has been applied to an electronic part such as a capacitor or a storage element. Furthermore, recently, a photovoltaic cell using a material having a perovskite type crystal structure has been developed.

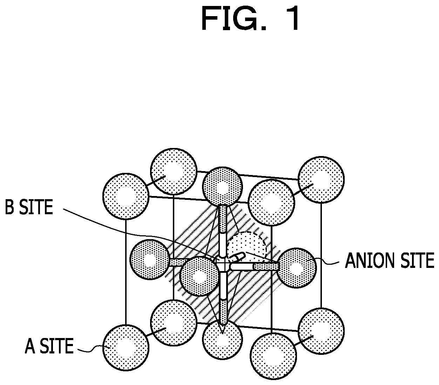

In the perovskite type crystal structure, cations are located at A site and B site, and an anion is located at an anion site (FIG. 1). Here, in order for the crystal to exist stably, the size of an ion at each site needs to satisfy a specific relationship. In addition, an amount representing geometric distortion of the perovskite type crystal structure is defined by the following tolerance factor (t) (see Non-Patent Document 1).

[Mathematical Formula 1]

$$t = \frac{(r_A + r_X)}{\sqrt{2}(r_B + r_X)}$$

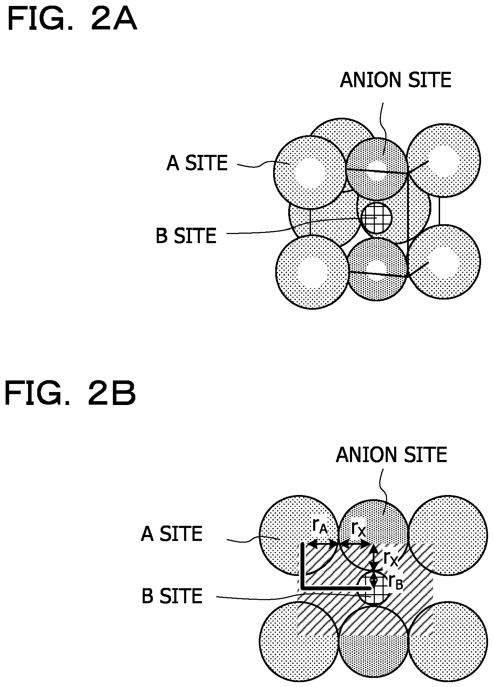

Here, $r_A$, $r_B$, and rx represent the ionic radii at A site, B site, and an anion site, respectively (FIGs. 2A and 2B). In addition, it is known that a tetragonal crystal is most stable when the tolerance factor (t) is 1.

Note that there is publicly known data of an ionic radius for each valence and coordination number of ionic species of most elements, and the publicly known data can be used (for example, see Non-Patent Documents 2 and 3).

As described above, the perovskite type crystal structure is a material whose physical properties largely change depending on a composition. Furthermore, in the perovskite type crystal structure, ions located at A site, B site, and an anion site can be replaced with various ions, and physical properties can be adjusted by forming a solid solution (or mixed crystal) of a plurality of perovskite materials. Specifically, for example, lead zirconate titanate (PZT) which is a typical ferroelectric material is obtained by replacing a part of B site of lead titanate in which a lead ion is located at A site, a titanium ion is located at B site, and an oxygen ion is located at an anion site with a zircon ion. As a method for calculating a tolerance factor in such a solid solution system, a method using a geometric mean weighted by a composition ratio to the ionic radius at a partially replaced site has been proposed.

The following are disclosed as related art:

Non-Patent Document 1: J. B. Goodenough and J. M. Longo, Landolt-Bornstein, Mew Series, Group. III, Vol. 4a, Springer-Verlag, Berlin, p.126 (1970),
Non-Patent Document 2: R. D. Shannon, Acta Crystallographica A32, p.751 (1976),
Non-Patent Document 3: Y. Q. Jia, Journal of Solid State Chemistry,95,184(1991),
Patent Document 1: CN 110021371 A,
Patent Document 2: US 2012/0180453 A1, and
Patent Document 3: US 2015/060743 A1.

Patent document 1 relates to a screening method of an organic-inorganic perovskite material. The screening method comprises the following steps: (1) an initial ABX3 organic-inorganic perovskite structure model is built; (2) atoms usually being tetravalent are selected to be candidate atoms, and a B site atom is replaced with other candidate atoms which can possibly form the stably available perovskite material; (3) a new structure after replacement is subjected to first principle calculation to complete structural relaxation; (4) the real stability of a convergence structure obtained after relaxation is evaluated through bond length and tolerance factors. The steps are repeated, and thus, the organic-inorganic perovskite material with a stable structure is screened out.

Patent document 2 relates to a method for catalytic oxidation of NO to $NO_2$ in the sulfur-containing exhaust gases of lean-burn engines, such as diesel engines. The catalysts are oxide perovskites with a credible likelihood of being sulfur-tolerant.

Patent document 3 relates to perovskite related compounds, which have layered structures in which perovskite units and A-rare earth structure units are alternately arranged. The reduced cell parameters $a_r$-$c_r$ and $\alpha_r$-$\gamma_r$ and the reduced cell volume $V_r$ are within the following ranges: $a_r$=6.05±0.6 Å, $b_r$=8.26±0.8 Å, $c_r$=9.10±0.9 Å, $\alpha_r$=103.4±10°, $\beta_r$=90±10°, $\gamma_r$=90±10°, and $V_r$=442.37±67 Å$^3$. At least one of the reduced cell parameters $a_r$-$c_r$ can be m/n times as large as the aforementioned values, where m and n are independent natural numbers, the square roots of 2 or 3 or integral multiples thereof. Values of $a_r$, $b_r$ and $c_r$ can be replaced with one another, or values of $\alpha_r$, $\beta_r$ and $\gamma_r$ can be replaced with one another.

However, in a case where there is a plurality of ionic species to be formed into a solid solution, and a combination and a composition of ionic species that make a tolerance factor 1 are determined on the basis thereof, calculation needs to be performed by changing the ionic species and the composition one by one. Then, in a case where the number of ionic species increases or in a case where a solid solution ratio is finely adjusted, the number of combinations is enormous, and the calculation takes a very long time.

It is desirable to provide a program capable of designing a composition of a stable perovskite type crystal structure at a high speed and a design method capable of designing a composition of a stable perovskite type crystal structure at a high speed.

SUMMARY

A design program causes a computer to execute a process for designing a composition of a perovskite type crystal structure, the design program comprising causing a computer to determine a combination of $A_i$ and $B_i$ in the following formula (2) created by taking a logarithm of a formula of a tolerance factor (t) represented by the following formula (1), in which logt is 0, by executing ground state search by an annealing method using an Ising model or QUBO,

$$t = \frac{(r_A + r_X)}{\sqrt{2}(r_B + r_X)} = \frac{d_{A-X}}{\sqrt{2}d_{B-X}} \qquad \text{... Formula (1)}$$

$$\log\ t = \sum_{i=1}^{n}A_i - \sum_{i=1}^{n}B_i \qquad \text{... Formula (2)}$$

wherein, in the formula (1), $r_A$, $r_B$, and $r_x$ represent ionic radii at A site, B site, and an anion site, respectively, when a general formula of the perovskite type crystal structure is represented by by ABX (in which A represents a cation, B represents a cation, and X represents an anion), and $d_{A-x}$ = $r_A$ + rx and $d_{B-x}$ = $r_B$ + rx are satisfied, in the formula (2), $A_i$ and $B_i$ are represented by the following formulas (3) and (4), respectively,

$$\frac{1}{n}\log\ d_{A_i-X} \equiv A_i \qquad \text{... Formula (3)}$$

$$\frac{1}{n}\log\ \sqrt{2}\,d_{B_i-X} \equiv B_i \qquad \text{... Formula (4)}$$

in the formulas (2), (3), and (4), n means n in $A^1_pA^2_q...B^1_rB^2_s...X_n$ (p + q + ... = r + s + ... = n; each number represents an integer) which is a composition formula when ions $A^1$, $A^2$, ..., and $A^u$ (composition ratio is p: q:...) are located at the A site, and ions $B^1$, $B^2$,..., and $B^v$ (composition ratio is r: s:...) are located at the B site in the ABX which is the general formula, and moreover, the $d_{A-x}$ and $d_{Ai-x}$ in the formula (3) satisfy the following formula (5), and the $d_{B-x}$ and $d_{Bi-x}$ in the formula (4) satisfy the following formula (6),

$$d_{A-X} = \left(\prod_{i=1}^{n}\left(r_{A_i} + r_X\right)\right)^{\frac{1}{n}} = \left(\prod_{i=1}^{n}d_{A_i-X}\right)^{\frac{1}{n}} \qquad \text{... Formula (5)}$$

$$d_{B-X} = \left(\prod_{i=1}^{n}(r_{B_i} + r_X)\right)^{\frac{1}{n}} = \left(\prod_{i=1}^{n} d_{B_i-X}\right)^{\frac{1}{n}} \quad \cdots \text{ Formula (6)}$$

in the formulas (5) and (6), n is the same as n in the formulas (2) to (4).

According to the disclosed design program, it is possible to solve the problems in related art and to provide a design program capable of designing a composition of a stable perovskite type crystal structure at a high speed.

According to the disclosed design method, it is possible to solve the problems in related art and to provide a design method capable of designing a composition of a stable perovskite type crystal structure at a high speed.

BRIEF DESCRIPTION OF DRAWINGS

Embodiments are set out, by way of example only, with reference to the following drawings, in which:

FIG. 1 is a schematic diagram illustrating an atomic arrangement of a perovskite type crystal structure;
FIG. 2A is a diagram for explaining a formula of a tolerance factor (No. 1);
FIG. 2B is a diagram for explaining a formula of a tolerance factor (No. 2);
FIG. 3 is a flowchart of an example of a disclosed design method;
FIG. 4 is a diagram illustrating a conceptual configuration of an optimizing device (arithmetic unit) used in an annealing method;
FIG. 5 is a circuit level block diagram of a transition control unit;
FIG. 6 is a diagram illustrating an operation flow of the transition control unit;
FIG. 7 is a configuration example of a disclosed design device;
FIG. 8 is another configuration example of the disclosed design device; and
FIG. 9 is another configuration example of the disclosed design device.

DESCRIPTION OF EMBODIMENTS

Regarding the perovskite type crystal structures illustrated in FIGs. 2A and 2B, typical elements and valences at A site and B site are illustrated in Table 1 below.

[Table 1]

| Site/Valence | Typical element | a | b |
|---|---|---|---|
| $A^+$ | Li, Na, K, Ag | 4 | |
| $A^{2+}$ | Pb, Ba, Sr, Ca | 4 | |
| $A^{3+}$ | Bi, La, Ce, Nd | 4 | |
| $B^+$ | Li, Cu | | 2 |
| $B^{2+}$ | Mg, Ni, Zn, Co, Sn, Fe, Cd, Cu, Cr | | 9 |
| $B^{3+}$ | Mn, Sb, Al, Yb, In, Fe, Co, Sc, Y, Sn | | 10 |
| $B^{4+}$ | Ti, Zr | | 2 |
| $B^{5*}$ | Nb, Sb, Ta, Bi | | 4 |
| $B^{6+}$ | W, Te, Re | | 3 |

In addition, a combination of cations of a perovskite type oxide or a perovskite type oxynitride and the number thereof, obtained by a simple combination in a case where only one element is located at each of A site and B site are, for example, illustrated in Table 2 below.

[Table 2]

| y | 0 | 1 | 2 | 3 |
|---|---|---|---|---|
| Composition | $O_3$ | $O_2N$ | $ON_2$ | $N_3$ |

(continued)

| y | 0 | | | 1 | | | 2 | | | 3 | | |
|---|---|---|---|---|---|---|---|---|---|---|---|---|
| Charge | -6 | | | -7 | | | -8 | | | -9 | | |
| $r_x$ (Å) | 1.35 | | | 1.39 | | | 1.42 | | | 1.46 | | |
| | | a | b | | a | b | | a | b | | a | b |
| Permissible combination of cations | $A^+ - B^{5+}$ | 4 | 4 | $A^+ - B^{6+}$ | 4 | 3 | $A^{2+} - B^{6+}$ | 4 | 3 | $A^{3+} - B^{6+}$ | 4 | 3 |
| | $A^{2+} - B^{4+}$ | 4 | 2 | $A^{2+} - B^{5+}$ | 4 | 4 | $A^{3+} - B^{5+}$ | 4 | 4 | | | |
| | $A^{3+} - B^{3+}$ | 4 | 10 | $A^{3+} - B^{4+}$ | 4 | 2 | | | | | | |

Here, in Tables 1 and 2, a represents the number of candidate ions at A site, and b represents the number of candidate ions at B site. In Tables 2 and 3 below, y represents y in an anion ($O_{3-y}N_y$). $r_x$ represents an ionic radius at an anion site.

In addition, the total number of permissible combinations of cations is 64 in a case of y = 0, 38 in a case of y = 1, 28 in a case of y = 2, and 12 in a case of y = 3, and the total number thereof is 142.

Therefore, in a case of a simple composition, calculation can be performed also by a method using a geometric mean for a composition in which the tolerance factor (t) is 1 or close to 1 in the following formula.

[Mathematical Formula 10]

$$t = \frac{(r_A + r_X)}{\sqrt{2}(r_B + r_X)} = \frac{d_{A-X}}{\sqrt{2}d_{B-X}}$$

Here, $r_A$, $r_B$, and rx represent the ionic radii at A site, B site, and an anion site, respectively. Note that $r_A + r_X$ = $d_A$-x and $r_B + r_X = d_{B-x}$ are satisfied in the rightmost side.

Then, for example, $d_A$-x and $d_B$-x are represented by the formulas in Table 3 below.

[Table 3]

| y | Composition formula | $d_{A-X}$ | $d_{B-X}$ |
|---|---|---|---|
| 0 | $ABO_3$ | $\left(\prod_{i=1}^{n}\left((r_{A_i}+r_O)^3\right)\right)^{\frac{1}{3n}} = \prod_{i=1}^{n}(r_{A_i}+r_O)^{\frac{1}{n}}$ | $\left(\prod_{i=1}^{n}\left((r_{B_i}+r_O)^3\right)\right)^{\frac{1}{3n}} = \prod_{i=1}^{n}(r_{B_i}+r_O)^{\frac{1}{n}}$ |
| 1 | $ABO_2N$ | $\left(\prod_{i=1}^{n}\left((r_{A_i}+r_O)^2(r_{A_i}+r_N)\right)\right)^{\frac{1}{3n}}$ | $\left(\prod_{i=1}^{n}\left((r_{B_i}+r_O)^2(r_{B_i}+r_N)\right)\right)^{\frac{1}{3n}}$ |
| 2 | $ABON_2$ | $\left(\prod_{i=1}^{n}\left((r_{A_i}+r_O)(r_{A_i}+r_N)^2\right)\right)^{\frac{1}{3n}}$ | $\left(\prod_{i=1}^{n}\left((r_{B_i}+r_O)(r_{B_i}+r_N)^2\right)\right)^{\frac{1}{3n}}$ |
| 3 | $ABN_3$ | $\left(\prod_{i=1}^{n}(r_{A_i}+r_N)^3\right)^{\frac{1}{3n}} = \prod_{i=1}^{n}(r_{A_i}+r_N)^{\frac{1}{n}}$ | $\left(\prod_{i=1}^{n}(r_{B_i}+r_N)^3\right)^{\frac{1}{3n}} = \prod_{i=1}^{n}(r_{B_i}+r_N)^{\frac{1}{n}}$ |

However, in a case where a plurality of elements is introduced into A site, B site, an anion site, or any combination thereof, and in a case where a solid solution ratio is finely adjusted, the number of combinations is enormous, and calculation takes a very long time.

Therefore, in the disclosed technology, by taking a logarithm of the formula of the tolerance factor (t), a stable combination of ions at A site, B site, and an anion site in the perovskite type crystal structure is determined by executing ground state search by an annealing method using an Ising model or quadratic unconstrained binary optimization (QUBO).

This makes it possible to perform calculation at a higher speed than a calculation method using a geometric mean even in a case where the number of combinations of ions at A site, B site, and an anion site is enormous.

(Design program for composition of perovskite type crystal structure and design method)

The disclosed design program is a design program for designing a composition of a perovskite type crystal structure, and includes causing a computer to determine a combination of $A_i$ and $B_i$ in the following formula (2) created by taking a logarithm of a formula of a tolerance factor (t) represented by the following formula (1), in which logt is 0 or close to 0, by executing ground state search by an annealing method using an Ising model or QUBO.

The disclosed design method is a design method for designing a composition of a perovskite type crystal structure using a computer, and includes determining a combination of $A_i$ and $B_i$ in the following formula (2) created by taking a logarithm of a formula of a tolerance factor (t) represented by the following formula (1), in which logt is 0 or close to 0, by executing ground state search by an annealing method using an Ising model or QUBO.

[Mathematical Formula 11]

$$t = \frac{(r_A + r_X)}{\sqrt{2}(r_B + r_X)} = \frac{d_{A-X}}{\sqrt{2}d_{B-X}} \quad \ldots \text{ Formula (1)}$$

[Mathematical Formula 12]

$$\log \ t = \sum_{i=1}^{n} A_i - \sum_{i=1}^{n} B_i \quad \ldots \text{ Formula (2)}$$

In formula (1), $r_A$, $r_B$, and $r_X$ represent ionic radii at A site, B site, and an anion site, respectively, when a general formula of a perovskite type crystal structure is represented by ABX (in which A represents a cation, B represents a cation, and X represents an anion), and $d_{A}$-x $= r_A + r_X$ and $d_{B-X} = r_B + r_X$ are satisfied.

In formula (2), $A_i$ and $B_i$ are represented by the following formulas (3) and (4), respectively.

[Mathematical Formula 13]

$$\frac{1}{n}\log \ d_{A_i - X} \equiv A_i \quad \ldots \text{ Formula (3)}$$

$$\frac{1}{n}\log \ \sqrt{2}\,d_{B_i - X} \equiv B_i \quad \ldots \text{ Formula (4)}$$

In formulas (2), (3), and (4), n means the parameter n in $A^1_p A^2_q ... B^1_r B^2_s ... X_n$ (p + q + ... = r + s + ... = n; each number represents an integer) which is a composition formula when ions $A^1$, $A^2$,..., and $A^u$ (composition ratio is p: q:...) are located at A site, and ions $B^1$, $B^2$,..., and $B^v$ (composition ratio is r: s:...) are located at B site in ABX which is a general formula. Moreover, $d_{A-X}$ and $d_{A_i-X}$ in formula (3) satisfy the following formula (5), and $d_{B}$-x and $d_{B_i-X}$ in formula (4) satisfy the following formula (6).

[Mathematical Formula 14]

$$d_{A-X} = \left(\prod_{i=1}^{n}(r_{A_i} + r_X)\right)^{\frac{1}{n}} = \left(\prod_{i=1}^{n} d_{A_i-X}\right)^{\frac{1}{n}} \qquad \cdots \text{ Formula (5)}$$

$$d_{B-X} = \left(\prod_{i=1}^{n}(r_{B_i} + r_X)\right)^{\frac{1}{n}} = \left(\prod_{i=1}^{n} d_{B_i-X}\right)^{\frac{1}{n}} \qquad \cdots \text{ Formula (6)}$$

In formulas (5) and (6), n is the same as n in formulas (2) to (4).

In the design program and the design method, for example, the kind of ion at A site and the kind of ion at B site are set to one or more specific ions, and ground state search is executed.

X (anion) in ABX is not particularly limited, can be appropriately selected depending on a purpose, and may be, for example, $O_{3-y}N_y$ (in which y represents an integer of 0 to 3) or a halogen.

When X represents $O_{3-y}N_y$, ABX is represented by $ABO_{3-y}N_y$ (in which y represents an integer of 0 to 3).

Generally, the Ising model is a model represented by the following energy function.

[Mathematical Formula 15]

$$H = \sum_{i \neq j} J_{ij}\sigma_i\sigma_j + \sum_{i} h_i\sigma_i \qquad (\sigma = \pm 1)$$

Here, $\sigma_i$ represents an input variable, and $\sigma \in \{-1, +1\}$ is satisfied. $J_{ij}$ is a (two-body) interaction parameter, and $h_i$ is referred to as a magnetic field as a (one-body) parameter. Note that a sign may be minus.

Here, input to an annealing machine that executes ground state search by an annealing method is performed by giving $J_{ij}$ and $h_i$. When these parameters are given, the machine executes annealing and outputs an approximate solution of a combination $\sigma$ that minimizes energy.

Furthermore, QUBO is a model in which a variable has been converted into $q \in \{0, 1\}$ which is compatible with bits instead of $\sigma \in \{-1, +1\}$.

The Ising model and QUBO are equivalent to each other because QUBO is obtained only by converting a variable in the Ising model [$\sigma = 2q - 1$ or $q = (\sigma + 1)/2$].

An example of the disclosed design method will be described with reference to a flowchart.

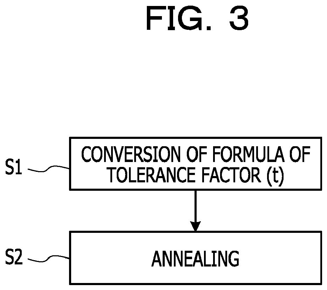

FIG. 3 is a flowchart of an example of the design method.

In an example of the design method, first, a formula of a tolerance factor (t) is converted (S1). The conversion is performed by taking a logarithm of the formula of the tolerance factor (t) represented by formula (1), and formula (2) is created after the conversion.

Next, a combination of $A_i$ and $B_i$ in formula (2), in which logt is 0 or close to 0, is determined by executing ground state search by an annealing method using the Ising model or QUBO (S2).

An example of a method for deriving formula (2) from formula (1) of the tolerance factor (t) will be described below. In the following example, an anion (X) is "$O_{3-y}N_y$".

The tolerance factor (t) is represented by the following formula.

[Mathematical Formula 16]

$$t = \frac{(r_A + r_X)}{\sqrt{2}(r_B + r_X)}$$

Here, $r_A$, $r_B$, and $r_X$ represent the ionic radii at A site, B site, and an anion site, respectively. There is publicly known data of an ionic radius for each valence and coordination number of ionic species of most elements, and the publicly known data can be used. Examples of the publicly known data include publicly known data disclosed in Non-Patent Documents 2 and 3.

The data of Non-Patent Document 2 can be easily obtained from http://research.kek.jp/people/hironori/nakao/lab/info/ionra dii-ele.html, http://pmsl.planet.sci.kobe-u.ac.jp/~seto/?page_id=51&lang=ja, and the like.

The data of Non-Patent Document 3 can be easily obtained from https://supercon.nims.go.jp/matprop/radion.html and the like.

Then, assuming that $r_A + r_X = d_{A-X}$ and $r_B + r_X = d_{B-X}$ are satisfied, the tolerance factor (t) is represented by the following formula.

[Mathematical Formula 17]

$$t = \frac{d_{A-X}}{\sqrt{2}d_{B-X}}$$

Then, in $A^1_p A^2_q ... B^1_r B^2_s ... O_{n(3-y)} N_{ny}$ (p + q + ... = r + s + ... = n; each number represents an integer) which is a composition formula when ions $A^1$, $A^2$,..., and $A^u$ (composition ratio is p: q:...) are located at A site, and ions $B^1$, $B^2$,..., and $B^v$ (composition ratio is r: s:...) are located at B site in a perovskite type crystal structure represented by a general formula $ABO_{3-y}N_y$, a geometric mean of each of compositions of $d_{A-X}$ and $d_{B-X}$ is represented by the following.

[Mathematical Formula 18]

$$d_{A-X} = \left(\prod_{i=1}^{n}\left(r_{A_i} + r_X\right)\right)^{\frac{1}{n}} = \left(\prod_{i=1}^{n}d_{A_i-X}\right)^{\frac{1}{n}}$$

$$d_{B-X} = \left(\prod_{i=1}^{n}\left(r_{B_i} + r_X\right)\right)^{\frac{1}{n}} = \left(\prod_{i=1}^{n}d_{B_i-X}\right)^{\frac{1}{n}}$$

In the formulas, $A_i$ means selecting p $A^1$s and q $A^2$s from {$A^1$, $A^2$...}. $B_i$ means selecting r $B^1$s and s $B^2$s from {$B^1$, $B^2$...}.

Based on the above description, the tolerance factor (t) is represented by the following formula.

[Mathematical Formula 19]

$$t = \frac{d_{A-X}}{\sqrt{2}d_{B-X}} = \frac{\left(\prod_{i=1}^{n}d_{A_i-X}\right)^{\frac{1}{n}}}{\sqrt{2}\left(\prod_{i=1}^{n}d_{B_i-X}\right)^{\frac{1}{n}}}$$

Here, in order to determine a stable perovskite type crystal structure, a composition of $A_i$ and $B_i$ in which t is 1 or close to 1 is determined.

However, it is difficult to handle the above formula in calculation of an annealing method for performing ground state search for an energy function represented by the Ising model.

Therefore, a logarithm of the above formula is taken, and an infinite product is converted into a total sum as follows.

[Mathematical Formula 20]

$$\log \ t = \log \ \frac{d_{A-X}}{\sqrt{2}d_{B-X}} = \log \ d_{A-X} - \log \ \sqrt{2}d_{B-X}$$

$$= \log \ \left(\prod_{i=1}^{n} d_{A_i-X}\right)^{\frac{1}{n}} - \log \ \sqrt{2}\left(\prod_{i=1}^{n} d_{B_i-X}\right)^{\frac{1}{n}}$$

$$= \frac{1}{n}\sum_{i=1}^{n} \log \ d_{A_i-X} - \frac{1}{n}\sum_{i=1}^{n} \log \ \sqrt{2}d_{B_i-X}$$

$$= \sum_{i=1}^{n}\frac{1}{n} \log \ d_{A_i-X} - \sum_{i=1}^{n}\frac{1}{n} \log \ \sqrt{2}d_{B_i-X}$$

Here, logt is represented by the following formula (2) when definition is made as represented by the following formulas (3) and (4).

[Mathematical Formula 21]

$$\frac{1}{n} \log \ d_{A_i-X} \equiv A_i \qquad \text{... Formula (3)}$$

$$\frac{1}{n} \log \ \sqrt{2} \ d_{B_i-X} \equiv B_i \qquad \text{... Formula (4)}$$

[Mathematical Formula 22]

$$\log \ t = \sum_{i=1}^{n} A_i - \sum_{i=1}^{n} B_i \qquad \text{... Formula (2)}$$

As described above, formula (2) can be derived from formula (1) of the tolerance factor (t).

Then, by setting $A_i$ and $B_i$ in a table and finding a combination of $A_i$ and $B_i$ in which logt is 0 or close to 0 by calculation of an annealing method, a composition of a stable perovskite type crystal structure can be determined.

Note that in order to determine a value at which logt is 0 or close to 0, the bottom of log may be 10 or e without any particular limitation.

Next, as one specific example, a method for determining a stable structure of a perovskite type oxide will be described.

As a prerequisite, a structure to be determined is an $ABO_3$ type perovskite type oxide. Furthermore, a combination of $A^+$ and $B^{5+}$ in Table 1 is used. In this case, a = 4 and b = 4 are satisfied, and a sum (m) of the number of candidate ions at A site (a) and the number of candidate ions at B site (b) is a + b = 8.

Furthermore, as a prerequisite, the composition is changed by 1/10 = 0.1 (n = p + q + ... = r + s + ... = 10). Therefore, when $ABO_3$ is $A_{10}B_{10}O_{30}$, the combination of $A^+$ and $B^{5+}$ can be represented as follows.

[Chemical Formula 1]        $Li_{0\sim10}Na_{0\sim10}K_{0\sim10}Ag_{0\sim10}Nb_{0\sim10}Sb_{0\sim10}Ta_{0\sim10}Bi_{0\sim10}O_{30}$

In this case, a matrix for optimization is illustrated in Table 4 below in which each column corresponds to each ionic species, and each of rows 1 to 10 represent a composition ratio.

[Table 4]

| j/i | Li 1 | Na 2 | K 3 | Ag 4 | Nb 5 | Sb 6 | Ta 7 | Bi 8 | = m |
|---|---|---|---|---|---|---|---|---|---|
| 1 | $X_{1,1}$ | $X_{2,1}$ | $X_{3,1}$ | $X_{4,1}$ | $X_{5,1}$ | $X_{6,1}$ | $X_{7,1}$ | $X_{8,1}$ | |
| 2 | $X_{1,2}$ | $X_{2,2}$ | $X_{3,2}$ | $X_{4,2}$ | $X_{5,2}$ | $X_{6,2}$ | $X_{7,2}$ | $X_{8,2}$ | |
| 3 | $X_{1,3}$ | $X_{2,3}$ | $X_{3,3}$ | $X_{4,3}$ | $X_{5,3}$ | $X_{6,3}$ | $X_{7,3}$ | $X_{8,3}$ | |
| 4 | $X_{1,4}$ | $X_{2,4}$ | $X_{3,4}$ | $X_{4,4}$ | $X_{5,4}$ | $X_{6,4}$ | $X_{7,4}$ | $X_{8,4}$ | |
| 5 | $X_{1,5}$ | $X_{2,5}$ | $X_{3,5}$ | $X_{4,5}$ | $X_{5,5}$ | $X_{6,5}$ | $X_{7,5}$ | $X_{8,5}$ | |
| 6 | $X_{1,6}$ | $X_{2,6}$ | $X_{3,6}$ | $X_{4,6}$ | $X_{5,6}$ | $X_{6,6}$ | $X_{7,6}$ | $X_{8,6}$ | |
| 7 | $X_{1,7}$ | $X_{2,7}$ | $X_{3,7}$ | $X_{4,7}$ | $X_{5,7}$ | $X_{6,7}$ | $X_{7,7}$ | $X_{8,7}$ | |
| 8 | $X_{1,8}$ | $X_{2,8}$ | $X_{3,8}$ | $X_{4,8}$ | $X_{5,8}$ | $X_{6,8}$ | $X_{7,8}$ | $X_{8,8}$ | |
| 9 | $X_{1,9}$ | $X_{2,9}$ | $X_{3,9}$ | $X_{4,9}$ | $X_{5,9}$ | $X_{6,9}$ | $X_{7,9}$ | $X_{8,9}$ | |
| n = 10 | $X_{1,10}$ | $X_{2,10}$ | $X_{3,10}$ | $X_{4,10}$ | $X_{5,10}$ | $X_{6,10}$ | $X_{7,10}$ | $X_{8,10}$ | |

Composition of each ion

Here, regarding $x_{i,j}$ = {0,1}, a composition (j) of each ion (i) is "1". For example, in a case of $Li_1Na_5K_4Nb_7Ta_3O_{30}$, only the hatched parts in Table 4 ($x_{1,1}$, $x_{2,5}$, $x_{3,4}$, $x_{5,7}$, and $x_{7,3}$) are "1", and the other parts are "0".

Meanwhile, Table 5 is obtained by applying the following formulas (3) and (4) to Table 4. At this time, the sign of B site is made negative in consideration of formula (2). Note that the values of Non-Patent Document 2 are used for each ion radii. Furthermore, in this calculation, the bottom of log is e.

[Mathematical Formula 23]

$$\frac{1}{n}\log \ d_{A_i-X} \equiv A_i \qquad \text{... Formula (3)}$$

$$\frac{1}{n}\log \ \sqrt{2}\, d_{B_i-X} \equiv B_i \qquad \text{... Formula (4)}$$

[Table 5]

| | $A_i$ | | | | $B_i$ | | | |
|---|---|---|---|---|---|---|---|---|
| | Li | Na | K | Ag | Nb | Sb | Ta | Bi |
| j\i | 1 | 2 | 3 | 4 | 5 | 6 | 7 | 8 |
| 1 | 0.0880 | 0.1058 | 0.1141 | 0.1019 | -0.1103 | -0.1084 | -0.1103 | -0.1158 |
| 2 | 0.1759 | 0.2116 | 0.2282 | 0.2038 | -0.2205 | -0.2167 | -0.2205 | -0.2315 |
| 3 | 0.2639 | 0.3173 | 0.3423 | 0.3057 | -0.3308 | -0.3251 | -0.3308 | -0.3473 |
| 4 | 0.3519 | 0.4231 | 0.4564 | 0.4075 | -0.4411 | -0.4335 | -0.4411 | -0.4630 |
| 5 | 0.4398 | 0.5289 | 0.5705 | 0.5094 | -0.5513 | -0.5419 | -0.5513 | -0.5788 |
| 6 | 0.5278 | 0.6347 | 0.6846 | 0.6113 | -0.6616 | -0.6502 | -0.6616 | -0.6945 |
| 7 | 0.6157 | 0.7405 | 0.7987 | 0.7132 | -0.7719 | -0.7586 | -0.7719 | -0.8103 |
| 8 | 0.7037 | 0.8462 | 0.9128 | 0.8151 | -0.8822 | -0.8670 | -0.8822 | -0.9260 |
| 9 | 0.7917 | 0.9520 | 1.0269 | 0.9170 | -0.9924 | -0.9754 | -0.9924 | -1.0418 |
| 10 | 0.8796 | 1.0578 | 1.1410 | 1.0188 | -1.1027 | -1.0837 | -1.1027 | -1.1575 |

Then, an objective function is represented by the following formula.

[Mathematical Formula 24]

$$D_{i,j} = \left(A_{i,j}B_{i,j}\right) \qquad D = \left(\sum_{i=1}^{m}\sum_{j=1}^{n}D_{ij}\cdot x_{ij}\right)^{2} \to 0$$

In the above formula, the coefficient $D_{ij}$ is an array of numbers $A_i$ and $B_i$ calculated in advance from the logarithm of the ionic radius, and i and j represent the matrix in Table 5. Ground state search is performed such that the absolute value of D approaches 0.

Here, as a constraint term, a constraint term that at each ion (column i) illustrated in Table 6 below, only one bit $x_{i,j}$ representing a composition (j) is "1" at most is added to the overall energy function.

[Table 6]

| | | Li | Na | K | Ag | Nb | Sb | Ta | Bi | |
|---|---|---|---|---|---|---|---|---|---|---|
| | j\i | 1 | 2 | 3 | 4 | 5 | 6 | 7 | 8 | = m |
| | 1 | $x_{1,1}$ | $x_{2,1}$ | $x_{3,1}$ | $x_{4,1}$ | $x_{5,1}$ | $x_{6,1}$ | $x_{7,1}$ | $x_{8,1}$ | |
| | 2 | $x_{1,2}$ | $x_{2,2}$ | $x_{3,2}$ | $x_{4,2}$ | $x_{5,2}$ | $x_{6,2}$ | $x_{7,2}$ | $x_{8,2}$ | |
| | 3 | $x_{1,3}$ | $x_{2,3}$ | $x_{3,3}$ | $x_{4,3}$ | $x_{5,3}$ | $x_{6,3}$ | $x_{7,3}$ | $x_{8,3}$ | |
| | 4 | $x_{1,4}$ | $x_{2,4}$ | $x_{3,4}$ | $x_{4,4}$ | $x_{5,4}$ | $x_{6,4}$ | $x_{7,4}$ | $x_{8,4}$ | |
| | 5 | $x_{1,5}$ | $x_{2,5}$ | $x_{3,5}$ | $x_{4,5}$ | $x_{5,5}$ | $x_{6,5}$ | $x_{7,5}$ | $x_{8,5}$ | |
| | 6 | $x_{1,6}$ | $x_{2,6}$ | $x_{3,6}$ | $x_{4,6}$ | $x_{5,6}$ | $x_{6,6}$ | $x_{7,6}$ | $x_{8,6}$ | |
| | 7 | $x_{1,7}$ | $x_{2,7}$ | $x_{3,7}$ | $x_{4,7}$ | $x_{5,7}$ | $x_{6,7}$ | $x_{7,7}$ | $x_{8,7}$ | |
| | 8 | $x_{1,8}$ | $x_{2,8}$ | $x_{3,8}$ | $x_{4,8}$ | $x_{5,8}$ | $x_{6,8}$ | $x_{7,8}$ | $x_{8,8}$ | |
| | 9 | $x_{1,9}$ | $x_{2,9}$ | $x_{3,9}$ | $x_{4,9}$ | $x_{59}$ | $x_{6,9}$ | $x_{7,9}$ | $x_{8,9}$ | |
| n = | 10 | $x_{1,10}$ | $x_{2,10}$ | $x_{3,10}$ | $x_{4,10}$ | $x_{5,10}$ | $x_{6,10}$ | $x_{7,10}$ | $x_{8,10}$ | |
| | $F_{i,j} = \sum_{j=1}^{n}x_{i,j}$ | $F_{1,j}$ | $F_{2,j}$ | $F_{3,j}$ | $F_{4,j}$ | $F_{5,j}$ | $F_{6,j}$ | $F_{7,j}$ | $F_{8,j}$ | |

This constraint term F is represented by the following formula.

[Mathematical Formula 25]

$$\sum_{j=1}^{n}x_{i,j} = F_i \in \{0,1\} \qquad F = \sum_{i=1}^{m}\bigl(F_i(F_i - 1)\bigr) = 0$$

By formulating the constraint term as the constraint term F, it is guaranteed that there is at most one bit that is 1 in a certain ionic species column, and it can be interpreted that the bit represents a composition ratio of the ionic species.

Moreover, as a constraint term, a constraint term that the total number of cations at A site and B site is a predetermined value is added to the overall energy function.

This constraint term G is represented by the following formula.

[Mathematical Formula 26]

$$\sum_{i=1}^{m}\sum_{j=1}^{n}j\cdot x_{i,j} = (p + q + \cdots) + (r + s + \cdots) = n + n = 2n$$

$$G = \left(\left(\sum_{i=1}^{m}\sum_{j=1}^{n}j\cdot x_{i,j}\right) - 2n\right)^{2} = 0$$

By formulating the constraint term as the constraint term G, it is guaranteed that the total number of cations at A site and B site is the number of cations determined by a perovskite type crystal structure.

Moreover, as a constraint term, a constraint term that the number of cations at A site and the number of cations at B site illustrated in Table 7 below are the same as each other is added to the overall energy function.

[Table 7]

|  | A site | | | | B site | | | |
|---|---|---|---|---|---|---|---|---|
| $H_{i,j}$ | Li | Na | K | Ag | Nb | Sb | Ta | Bi |
| j\i | 1 | 2 | 3 | 4 | 5 | 6 | 7 | 8 |
| 1 | 1 | 1 | 1 | 1 | -1 | -1 | -1 | -1 |
| 2 | 2 | 2 | 2 | 2 | -2 | -2 | -2 | -2 |
| 3 | 3 | 3 | 3 | 3 | -3 | -3 | -3 | -3 |
| 4 | 4 | 4 | 4 | 4 | -4 | -4 | -4 | -4 |
| 5 | 5 | 5 | 5 | 5 | -5 | -5 | -5 | -5 |
| 6 | 6 | 6 | 6 | 6 | -6 | -6 | -6 | -6 |
| 7 | 7 | 7 | 7 | 7 | -7 | -7 | -7 | -7 |
| 8 | 8 | 8 | 8 | 8 | -8 | -8 | -8 | -8 |
| 9 | 9 | 9 | 9 | 9 | -9 | -9 | -9 | -9 |
| 10 | 10 | 10 | 10 | 10 | -10 | -10 | -10 | -10 |

This constraint term H is represented by the following formula.

[Mathematical Formula 27]

$$H = \left( \sum_{i=1}^{m} \sum_{j=1}^{n} H_{ij} \cdot x_{ij} \right)^2 = 0$$

Although the total number of cations is guaranteed by the constraint term G, it is not guaranteed that the number of cations at each of A site and B site is the number of cations determined by a perovskite type crystal structure. By adding the constraint term H, it is guaranteed that the number of cations at A site is the same as the number of cations at B site.

Here, as illustrated in Table 7, by using a numerical table in which the signs of the numbers representing the compositions at A site and B site are reversed to each other, an ionic species in which $x_{ij}$ is simply 1 and a total sum of the compositions are determined. When the total sum is 0, it is possible to guarantee that a condition that the number of cations at A site is the same as the number of cations at B site is satisfied.

The above-described objective function and constraint term are summarized as follows.

[Mathematical Formula 28]

Objective function $\quad D = \left( \sum_{i=1}^{m} \sum_{j=1}^{n} D_{ij} \cdot x_{ij} \right)^2 \to 0$

Constraint term
$$\begin{cases} F = \sum_{i=1}^{m} \left( F_i (F_i - 1) \right) = 0 \qquad F_i = \sum_{j=1}^{n} x_{i,j} \\ \\ G = \left( \left( \sum_{i=1}^{m} \sum_{j=1}^{n} j \cdot x_{i,j} \right) - 2n \right)^2 = 0 \\ \\ H = \left( \sum_{i=1}^{m} \sum_{j=1}^{n} H_{ij} \cdot x_{ij} \right)^2 = 0 \end{cases}$$

Note that the closer the objective function is to 0, the better. Furthermore, the constraint term is 0.

Then, the overall energy function (E) is represented as follows.

[Mathematical Formula 29]

Overall energy function $\qquad E = \alpha D + \beta F + \gamma G + \delta H$

Since all the terms D, F, G, and H each are 0 or more, E is a value of 0 or more.

Here, $\alpha$, $\beta$, $\gamma$, and $\delta$ are parameters that adjust a balance between the constraint term and the objective function, and are set appropriately.

Then, by determining a bit pattern of a matrix in Table 4 in which E is from a minimum value to 0 by annealing, it is possible to design ionic species that achieve a perovskite type oxide having a stable crystal structure containing Li, Na, K, Ag, or any combination thereof, and Nb, Sb, Ta, Bi, or any combination thereof, and a combination of compositions thereof at a high speed.

Note that as a prerequisite in a specific example, a structure to be determined is an $ABO_3$ type perovskite type oxide, and is moreover, a combination of $A^+$ and $B^{5+}$. Therefore, as a prerequisite in the above specific example, the charge is neutral.

Meanwhile, in a case where the charge does not become neutral only by the prerequisite, a constraint term that the charge becomes neutral is added to the overall energy function (E).

An annealing machine may be a quantum annealing machine, a semiconductor annealing machine using semiconductor technology, or simulated annealing executed by software using a central processing unit (CPU) or a graphics processing unit (GPU) as long as being a computer that adopts an annealing method that performs ground state search for an energy function represented by an Ising model.

Examples of the annealing method and the annealing machine will be described below.

The annealing method (simulated annealing method, SA method) is a kind of Monte Carlo method, and is a method for probabilistically determining a solution using a random number value. The following describes a problem of minimizing a value of an evaluation function to be optimized as an example. The value of the evaluation function is referred to as energy. For maximization, the sign of the evaluation function only needs to be changed.

A process is started from an initial state in which one of discrete values is assigned to each variable. With respect to a current state (combination of variable values), a state close to the current state (for example, a state in which only one variable is changed) is selected, and a state transition therebetween is considered. An energy change with respect to the state transition is calculated. Depending on the value, it is probabilistically determined whether to adopt the state transition to change the state or not to adopt the state transition to keep the original state. In a case where an adoption probability when the energy goes down is selected to be larger than that when the energy goes up, it can be expected that a state change will occur in a direction that the energy goes down on average, and that a state transition will occur to a more appropriate state over time. Then, finally, there is a possibility that an optimal solution or an approximate solution that gives energy close to the optimal value can be obtained. If this is adopted when the energy goes down deterministically and is not adopted when the energy goes up, the energy change decreases monotonically in a broad sense with respect to time, but no further change occurs when a local solution is reached. As described above, since there are a very a large number of local solutions in the discrete optimization problem, a state is almost certainly caught in a local solution that is not so close to an optimum value. Therefore, it is important to determine probabilistically whether to adopt a state transition.

In the annealing method, it has been proved that by determining an adoption (permissible) probability of a state transition as follows, a state reaches an optimum solution in the limit of infinite time (iteration count).

(A) For an energy change (energy reduction) value $(-\Delta E)$ due to a state transition, a permissible probability p of the state transition is determined by any one of the following functions f ().

[Mathematical Formula 30]

$$p(\Delta E, T) = f(-\Delta E / T) \qquad \text{Formula (A-1)}$$

[Mathematical Formula 31]

$$f_{metro}(x) = \min(1, e^x) \qquad \text{(Metropolis method)} \quad \text{Formula (A-2)}$$

[Mathematical Formula 32]

$$f_{Gibbs}(x) = \frac{1}{1+e^{-x}}$$

(Gibbs method)   Formula (A-3)

Here, T is a parameter called a temperature value and is changed as follows.
(B) The temperature value T is logarithmically reduced with respect to an iteration count t as represented by the following formula.
[Mathematical Formula 33]

$$T = \frac{T_0 \log(c)}{\log(t+c)}$$

Formula (B)

Here, $T_0$ is an initial temperature value, and is desirably a sufficiently large value depending on a problem.

In a case where the permissible probability represented by the formula described in (A) is used, if a steady state is reached after sufficient iterations, an occupation probability of each state follows a Boltzmann distribution for a thermal equilibrium state in thermodynamics. Then, when the temperature is gradually lowered from a high temperature, an occupation probability of a low energy state increases. Therefore, the low energy state may be obtained when the temperature is sufficiently lowered. Since this state is very similar to a state change caused when a material is annealed, this method is referred to as an annealing method (or pseudo-annealing method). At this time, probabilistic occurrence of a state transition that increases energy corresponds to thermal excitation in physics.

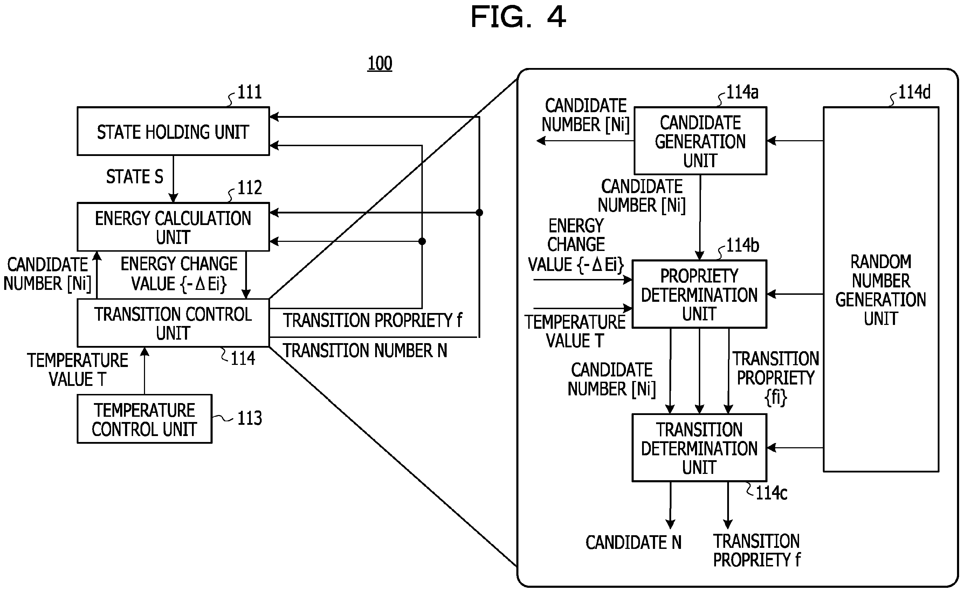

FIG. 4 illustrates a conceptual configuration of an optimizing device (arithmetic unit 18) that performs the annealing method. However, in the following description, a case of generating a plurality of state transition candidates is also described, but an original basic annealing method generates one transition candidate at a time.

First, an optimizing device 100 includes a state holding unit 111 that holds a current state S (a plurality of state variable values). Furthermore, the optimizing device 100 includes an energy calculation unit 112 that calculates an energy change value {-ΔEi} of each state transition when a state transition from the current state S occurs due to a change in any one of the plurality of state variable values. In addition, the optimizing device 100 includes a temperature control unit 113 that controls a temperature value T and a transition control unit 114 that controls a state change.

The transition control unit 114 probabilistically determines whether to accept or not any one of a plurality of state transitions according to a relative relationship between an energy change value {-ΔEi} and thermal excitation energy on the basis of a temperature value T, the energy change value {-ΔEi}, and a random value.

When the transition control unit 114 is further subdivided, the transition control unit 114 includes a candidate generation unit 114a that generates a state transition candidate, and a propriety determination unit 114b for probabilistically determining whether or not to permit a state transition for each candidate on the basis of an energy change value {-ΔEi} and a temperature value T. Moreover, the transition control unit 114 includes a transition determination unit 114c that determines a candidate to be adopted from the candidates that have been permitted, and a random number generation unit 114d that generates a random variable.

Operation in one iteration is as follows. First, the candidate generation unit 114a generates one or more state transition candidates (candidate number {Ni}) from the current state S held in the state holding unit 111 to a next state. The energy calculation unit 112 calculates an energy change value {-ΔEi} for each state transition listed as a candidate using the current state S and the state transition candidates. The propriety determination unit 114b permits a state transition with a permissible probability of the formula described in above (A) according to an energy change value {-ΔEi} of each state transition using the temperature value T generated by the temperature control unit 113 and the random variable (random number value) generated by the random number generation unit 114d. Then, the propriety determination unit 114b outputs propriety {fi} of each state transition. In a case where there is a plurality of permitted state transitions, the transition determination unit 114c randomly selects one of the permitted state transitions using a random number value. Then, the transition determination unit 114c outputs a transition number N of the selected state transition and transition propriety f. In a case where there is a permitted state transition, a state variable value stored in the state holding unit 111 is updated according to the adopted state transition.

Starting from an initial state, the above-described iteration is repeated while the temperature value is lowered by the temperature control unit 113. When a completion determination condition such as reaching a certain iteration count or energy falling below a certain value is satisfied, the operation is completed. An answer output by the optimizing device 110 is a state when the operation is completed.

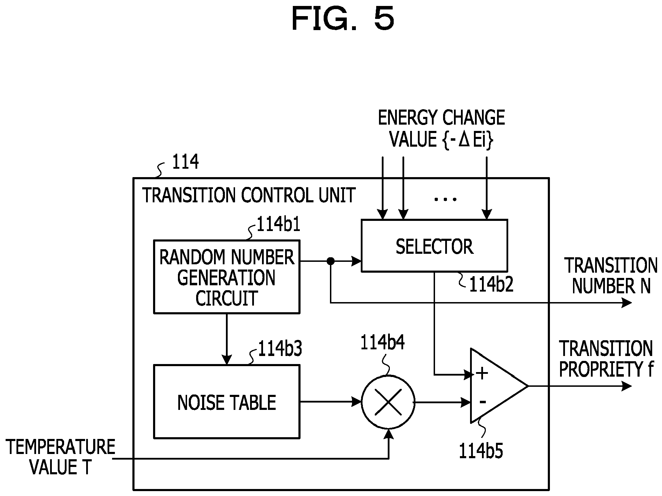

FIG. 5 is a circuit-level block diagram of a configuration example of a transition control unit in a normal annealing method for generating one candidate at a time, particularly an arithmetic unit for a propriety determination unit.

The transition control unit 114 includes a random number generation circuit 114b1, a selector 114b2, a noise table 114b3, a multiplier 114b4, and a comparator 114b5.

The selector 114b2 selects and outputs a value corresponding to the transition number N which is a random number value generated by the random number generation circuit 114b1 among energy change values {-$\Delta E_i$} calculated for each state transition candidates.

The function of the noise table 114b3 will be described later. As the noise table 114b3, for example, a memory such as a random access memory (RAM) or a flash memory can be used.

The multiplier 114b4 outputs a product obtained by multiplying a value output by the noise table 114b3 by a temperature value T (corresponding to the above-described thermal excitation energy).

The comparator 114b5 outputs a comparison result obtained by comparing a multiplication result output by the multiplier 114b4 with -$\Delta E$ which is an energy change value selected by the selector 114b2 as transition propriety f.

The transition control unit 114 illustrated in FIG. 5 basically implements the above-described functions as they are. However, a mechanism that permits a state transition with a permissible probability represented by the formula described in (A) has not been described so far, and therefore will be described.

A circuit that outputs 1 at a permissible probability p and outputs 0 at a permissible probability (1-p) can be achieved by inputting a uniform random number that takes the permissible probability p for input A and takes a value of an interval [0, 1) for input B in a comparator that has two inputs A and B, outputs 1 when A > B is satisfied and outputs 0 when A < B is satisfied. Therefore, if a value of a permissible probability p calculated on the basis of the energy change value and the temperature value T using the formula described in (A) is input to input A of this comparator, the above-described function can be achieved.

That is, with a circuit that outputs 1 when f ($\Delta E/T$) is larger than u, in which f is a function used in the formula described in (A), and u is a uniform random number that takes a value of an interval [0, 1), the above-described function can be achieved.

Although it may be left as it is, the same function can be achieved also by the following modification. Applying the same monotonically increasing function to two numbers does not change the magnitude relationship. Therefore, an output is not changed even if the same monotonically increasing function is applied to two inputs of the comparator. If an inverse function $f^{-1}$ of f is adopted as this monotonically increasing function, it can be found that a circuit that outputs 1 when -$\Delta E/T$ is larger than $f^{-1}$ (u) may be sufficient. Moreover, since the temperature value T is positive, a circuit that outputs 1 when -$\Delta E$ is larger than $Tf^{-1}(u)$ may be sufficient. The noise table 114b3 in FIG. 5 is a conversion table for achieving this inverse function $f^{-1}(u)$, and is a table that outputs a value of the following function to an input that discretizes the interval [0,1).

[Mathematical Formula 34]

$$f_{metro}^{-1}(u) = \log(u) \qquad \text{Formula (C-1)}$$

[Mathematical Formula 35]

$$f_{Gibbs}^{-1}(u) = \log\left(\frac{u}{1-u}\right) \qquad \text{Formula (C-2)}$$

The transition control unit 114 also includes a latch that holds a determination result and the like, a state machine that generates a timing thereof, and the like, but these are not illustrated in FIG. 5 for simplicity of illustration.

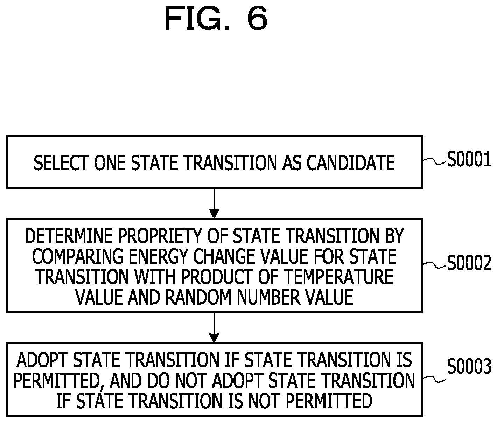

FIG. 6 is an operation flow of the transition control unit 114. The operation flow includes a step of selecting one state transition as a candidate (S0001), a step of determining propriety of the state transition by comparing an energy change value for the state transition with a product of a temperature value and a random number value (S0002), and a step of adopting the state transition if the state transition is permitted, and not adopting the state transition if the state transition is not permitted (S0003).

A program can be created using various known programming languages according to the configuration of a computer system used, the kind and version of an operating system, and the like.

The program may be recorded on a recording medium such as an internal hard disk or an external hard disk, or may be recorded on a recording medium such as a compact disc read only memory (CD-ROM), a digital versatile disk read only memory (DVD-ROM), a magneto-optical (MO) disk, or a universal serial bus (USB) memory [USB flash drive], for example. In a case where the program is recorded on a recording medium such as a CD-ROM, a DVD-ROM, an MO disk, or a USB memory, the program can be directly used through a recording medium reader included in the computer system, or be installed into a hard disk and be then used, as needed. Alternatively, the program can be recorded in an external storage area (another computer or the like) that is accessible from the computer system through an information communication network, and this program can be directly used from the external storage area through an information communication network, or be installed into a hard disk and then be used, as needed.

The program may be divided into respective arbitrary processes, and be recorded on a plurality of recording media.

Recording medium

A recording medium disclosed in the present case records the design program disclosed in the present case.

The recording medium is computer-readable.

The recording medium disclosed in the present case is not particularly limited, and can be appropriately selected according to a purpose. Examples of the recording medium include an internal hard disk, an external hard disk, a CD-ROM, a DVD-ROM, an MO disk, and a USB memory.

Furthermore, the recording medium disclosed in the present case may be a plurality of recording media on which the design program disclosed in the present case is divided into respective arbitrary processes, and is recorded.

The recording medium may be transitory or non-transitory.

(Design device)

The disclosed design device includes at least an execution unit, and further includes another unit as needed.

The disclosed design device designs a composition of a perovskite type crystal structure.

The execution unit determines a combination of $A_i$ and $B_i$ in formula (2) created by taking a logarithm of a formula of a tolerance factor (t) represented by formula (1), in which logt is 0 or close to 0, by executing ground state search by an annealing method using an Ising model or QUBO.

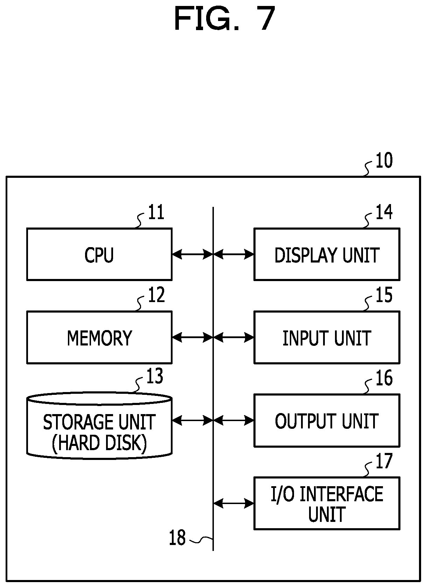

FIG. 7 illustrates a configuration example of the disclosed design device.

A design device 10 includes a CPU 11, a memory 12, a storage unit 13, a display unit 14, an input unit 15, an output unit 16, an I/O interface unit 17, and the like that are connected to each other via a system bus 18, for example.

The CPU 11 performs arithmetic operations (such as four arithmetic operations and comparison operations), hardware and software operation control, and the like.

The memory 12 is a memory such as a random access memory (RAM) or a read only memory (ROM). The RAM stores an operating system (OS), an application program, and the like read from the ROM and the storage unit 13, and functions as a main memory and a work area of the CPU 11.

The storage unit 13 is a device that stores various kinds of programs and data, and may be a hard disk, for example. The storage unit 13 stores a program to be executed by the CPU 11, data to be used in executing the program, an OS, and the like.

A program is stored in the storage unit 13, is loaded into the RAM (main memory) of the memory 12, and is executed by the CPU 11.

The display unit 14 is a display device, and may be a display device such as a CRT monitor or a liquid crystal panel, for example.

The input unit 15 is an input device for various kinds of data, and may be a keyboard, a pointing device (such as a mouse), or the like, for example.

The output unit 16 is an output device for various kinds of data, and may be a printer, for example.

The I/O interface unit 17 is an interface for connecting various external devices. For example, the I/O interface unit 17 enables inputting/outputting of data into/from a CD-ROM, a DVD-ROM, an MO disk, a USB memory, or the like.

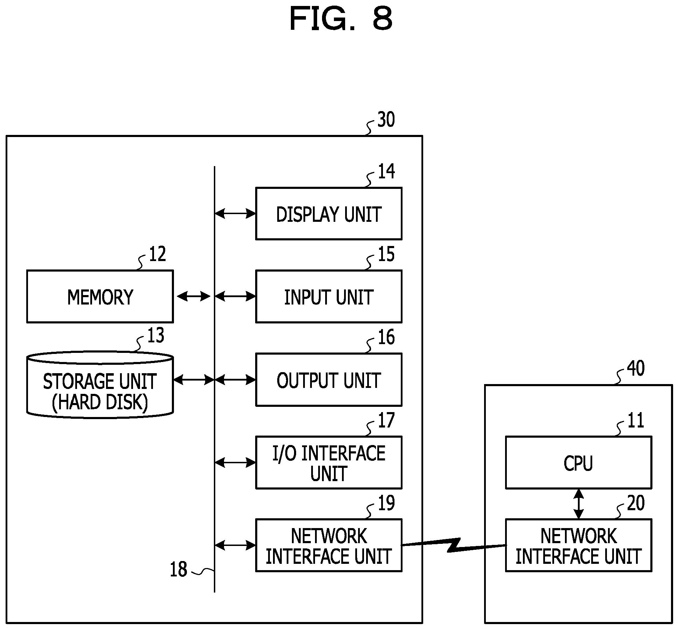

FIG. 8 illustrates another configuration example of the disclosed design device.

The configuration example in FIG. 8 is a cloud type configuration example, in which the CPU 11 is independent of the storage unit 13 and the like. In this configuration example, a computer 30 that includes the storage unit 13 and the like is connected to a computer 40 that includes the CPU 11 via network interface units 19 and 20.

The network interface units 19 and 20 are hardware that performs communication using the Internet.

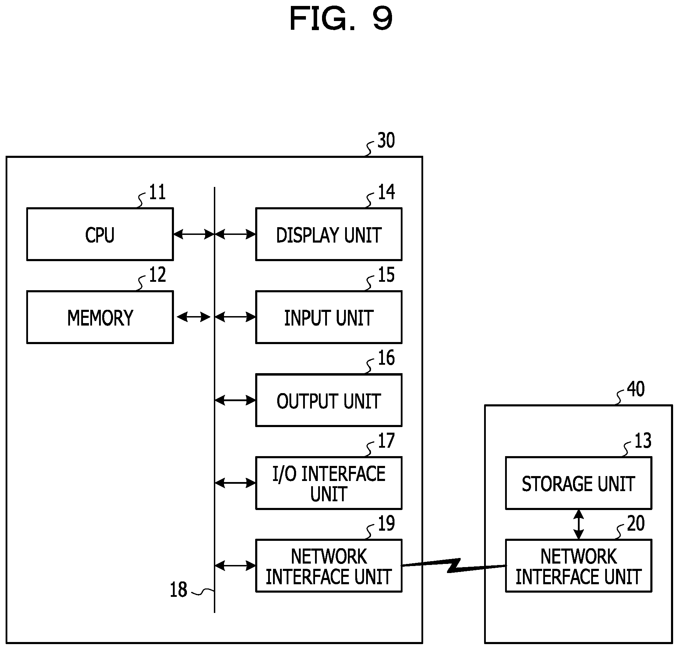

FIG. 9 illustrates another configuration example of the disclosed design device.

The configuration example in FIG. 9 is a cloud type configuration example, in which the storage unit 13 is independent of the CPU 11 and the like. In this configuration example, a computer 30 that includes the CPU 11 and the like is connected to a computer 40 that includes the storage unit 13 via the network interface units 19 and 20.

In any of the above aspects, the various features may be implemented in hardware, or as software modules running on one or more processors/computers.

The invention also provides a computer program or a computer program product comprising instructions which, when executed by a computer, cause the computer to carry out any of the methods/method steps described herein, and a non-transitory computer-readable medium comprising instructions which, when executed by a computer, cause the computer to carry out any of the methods/method steps described herein. A computer program embodying the invention may be stored on a non-transitory computer-readable medium, or it could, for example, be in the form of a signal such as a downloadable data signal provided from an Internet website, or it could be in any other form.

**Claims**

1. A design program for causing a computer to execute a process for designing a composition of a perovskite type crystal structure, the design program comprising causing a computer to determine a combination of $A_i$ and $B_i$ in the following formula (2) created by taking a logarithm of a formula of a tolerance factor, t, represented by the following formula (1), in which logt is 0, by executing ground state search by an annealing method using an Ising model or QUBO,

$$t = \frac{(r_A + r_X)}{\sqrt{2}(r_B + r_X)} = \frac{d_{A-X}}{\sqrt{2}d_{B-X}} \quad \ldots \text{Formula (1)}$$

$$\log \; t = \sum_{i=1}^{n} A_i - \sum_{i=1}^{n} B_i \quad \ldots \text{Formula (2)}$$

wherein, in the formula (1), $r_A$, $r_B$, and rx represent ionic radii at A site, B site, and an anion site, respectively, when a general formula of the perovskite type crystal structure is represented by ABX, in which A represents a cation, B represents a cation, and X represents an anion, and $d_{A-X} = r_A + rx$ and $d_{B-X} = r_B + rx$ are satisfied, wherein, in the formula (2), $A_i$ and $B_i$ are represented by the following formulas (3) and (4), respectively,

$$\frac{1}{n}\log \; d_{A_i-X} \equiv A_i \quad \ldots \text{Formula (3)}$$

$$\frac{1}{n}\log \; \sqrt{2}\, d_{B_i-X} \equiv B_i \quad \ldots \text{Formula (4)}$$

wherein, in the formulas (2), (3), and (4), n is the parameter n in a composition formula $A^1_p A^2_q...B^1_r B^2_s...X_n$, where p + q + ... = r + s + ... = n and each number represents an integer, and where ions $A^1$, $A^2$, ..., and $A^u$ of composition ratio p: q: ... are located at the A site, and ions $B^1$, $B^2$,..., and $B^v$ of composition ratio r: s:... are located at the B site in the ABX which is the general formula, and moreover, the $d_{A-X}$ and $d_{Ai-X}$ in the formula (3) satisfy the following formula (5), and the $d_B$-x and $d_{Bi-X}$ in the formula (4) satisfy the following formula (6),

$$d_{A-X} = \left(\prod_{i=1}^{n}\left(r_{A_i} + r_X\right)\right)^{\frac{1}{n}} = \left(\prod_{i=1}^{n} d_{A_i-X}\right)^{\frac{1}{n}} \quad \ldots \text{Formula (5)}$$

$$d_{B-X} = \left(\prod_{i=1}^{n}\left(r_{B_i} + r_X\right)\right)^{\frac{1}{n}} = \left(\prod_{i=1}^{n} d_{B_i-X}\right)^{\frac{1}{n}} \quad \ldots \text{Formula (6)}$$

wherein, in the formulas (5) and (6), n is the same as n in the formulas (2) to (4).

2. The design program according to claim 1, wherein the kind of ion at the A site and the kind of ion at the B site are set to one or more specific ions, and the ground state search is executed.

3. The design program according to claim 1 or 2, wherein the ABX is represented by $ABO_{3-y}N_y$, in which y represents an integer of 0 to 3.

4. A computer implemented design method for designing a composition of a perovskite type crystal structure using a computer, the computer implemented design method comprising

determining a combination of $A_i$ and $B_i$ in the following formula (2) created by taking a logarithm of a formula of a tolerance factor, t, represented by the following formula (1), in which logt is 0, by executing ground state search by an annealing method using an Ising model or QUBO,

$$t = \frac{(r_A + r_X)}{\sqrt{2}(r_B + r_X)} = \frac{d_{A-X}}{\sqrt{2}d_{B-X}} \qquad \ldots \texttt{Formula (1)}$$

$$\log\ t = \sum_{i=1}^{n} A_i - \sum_{i=1}^{n} B_i \qquad \ldots \texttt{Formula (2)}$$

wherein, in the formula (1), $r_A$, $r_B$, and rx represent ionic radii at A site, B site, and an anion site, respectively, when a general formula of the perovskite type crystal structure is represented by ABX, in which A represents a cation, B represents a cation, and X represents an anion, and $d_{A-X} = r_A + rx$ and $d_{B-X} = r_B + rx$ are satisfied, wherein in the formula (2), $A_i$ and $B_i$ are represented by the following formulas (3) and (4), respectively,

$$\frac{1}{n}\log\ d_{A_i-X} \equiv A_i \qquad \ldots \texttt{Formula (3)}$$

$$\frac{1}{n}\log\ \sqrt{2}\,d_{B_i-X} \equiv B_i \ \ldots \texttt{Formula (4)}$$

wherein, in the formulas (2), (3), and (4), n is the parameter n in a composition formula $A^1_p A^2_q...B^1_r B^2_s...X_n$, where p + q + ... = r + s + ... = n and each number represents an integer, and where ions $A^1$, $A^2$, ..., and $A^u$ of composition ratio p: q: ... are located at the A site, and ions $B^1$, $B^2$,..., and $B^v$ of composition ratio r: s:... are located at the B site in the ABX which is the general formula, and moreover, the $d_{A-X}$ and $d_{Ai-X}$ in the formula (3) satisfy the following formula (5), and the $d_{B-X}$ and $d_{Bi-X}$ in the formula (4) satisfy the following formula (6),

$$d_{A-X} = \left(\prod_{i=1}^{n}(r_{A_i} + r_X)\right)^{\frac{1}{n}} = \left(\prod_{i=1}^{n} d_{A_i-X}\right)^{\frac{1}{n}} \quad \ldots \texttt{Formula (5)}$$

$$d_{B-X} = \left(\prod_{i=1}^{n}(r_{B_i} + r_X)\right)^{\frac{1}{n}} = \left(\prod_{i=1}^{n} d_{B_i-X}\right)^{\frac{1}{n}} \quad \ldots \texttt{Formula (6)}$$

wherein, in the formulas (5) and (6), n is the same as n in the formulas (2) to (4).

5. The computer implemented design method according to claim 4, wherein the kind of ion at the A site and the kind of ion at the B site are set to one or more specific ions, and the ground state search is executed.

6. The computer implemented design method according to claim 4 or 5, wherein the ABX is represented by $ABO_{3-y}N_y$, in which y represents an integer of 0 to 3.

**Patentansprüche**

1. Entwurfsprogramm zum Veranlassen eines Computers, einen Prozess zum Entwerfen einer Zusammensetzung einer Kristallstruktur vom Perowskit-Typ auszuführen, wobei das Entwurfsprogramm umfasst, einen Computer zu veranlassen, eine Kombination von $A_i$ und B; in der folgenden Formel (2) zu bestimmen, die durch Logarithmieren einer Formel eines Toleranzfaktors t erzeugt wird, dargestellt durch die folgende Formel (1), in der logt 10 ist, durch Ausführen einer Grundzustandssuche durch ein Temperverfahren unter Verwendung eines Ising-Modells oder QUBO,

$$t = \frac{(r_A + r_X)}{\sqrt{2}(r_B + r_X)} = \frac{d_{A-X}}{\sqrt{2}d_{B-X}} \text{ ... Formel (1)}$$

$$\log t = \sum_{i=1}^{n} A_i - \sum_{i=1}^{n} B_i \text{ ... Formel (2)}$$

wobei in Formel (1) $r_A$, $r_B$ und $r_X$ ionenradien an einer A-Stelle, B-Stelle bzw. einer Anionenstelle darstellen, wenn eine allgemeine Formel der Kristallstruktur vom Perowskit-Typ durch ABX dargestellt wird, wobei A ein Kation darstellt, B ein Kation darstellt und X ein Anion darstellt, und $d_{A-X} = r_A + r_X$ und $d_{B-X} = r_B + r_X$ erfüllt sind, wobei in der Formel (2) $A_i$ und $B_i$ jeweils durch die folgenden Formeln (3) und (4) dargestellt werden,

$$\frac{1}{n}\log d_{A_i-X} \equiv A_i \text{ ... Formel (3)}$$

$$\frac{1}{n}\log \sqrt{2}d_{B_i-X} \equiv B_i \text{ ... Formel (4)}$$

wobei in den Formeln (2), (3) und (4) n der Parameter n in einer Zusammensetzungsformel $A^1_p A^2_q ... B^1_r B^2_x ... X_n$ ist, wobei p + q + ...= r + s + ... = n ist und jede Zahl ein ganze Zahl darstellt, und wobei sich die Ionen $A^1$, $A^2$, ... und $A^u$ des Zusammensetzungsverhältnisses p: q: ... an der A-Stelle befinden, und die Ionen $B^1$, $B^2$, ... und $B^v$ des Zusammensetzungsverhältnisses r: s : ... sich an der B-Stelle befinden in ABX, was die allgemeine Formel ist, und darüber hinaus die $d_{A-X}$ und $d_{A_i-X}$ in der Formel (3) die folgende Formel (5) erfüllen, und die $d_{B-X}$ und $d_{B_i-X}$ in die Formel (4) die folgende Formel (6) erfüllen,

$$d_{A-X} = \left(\prod_{i=1}^{n} (r_{A_i} + r_X)\right)^{\frac{1}{n}} = \left(\prod_{i=1}^{n} d_{A_i-X}\right)^{\frac{1}{n}} \text{ ... Formel (5)}$$

$$d_{B-X} = \left(\prod_{i=1}^{n} (r_{B_i} + r_X)\right)^{\frac{1}{n}} = \left(\prod_{i=1}^{n} d_{B_i-X}\right)^{\frac{1}{n}} \text{ ... Formel (6)}$$

wobei in den Formeln (5) und (6) n gleiche wie das n in den Formeln (2) bis (4) ist.

2. Entwurfsprogramm nach Anspruch 1, wobei die Ionenart an der A-Stelle und die Ionenart an der B-Stelle auf eines oder mehrere spezifische Ionen eingestellt sind, und die Grundzustandssuche ausgeführt wird.

3. Entwurfsprogramm nach Anspruch 1 oder 2, wobei ABX durch $ABO_{3-y}N_y$ dargestellt wird, wobei y eine ganze Zahl von 0 bis 3 darstellt.

4. Computerimplementiertes Entwurfsverfahren zum Entwerfen einer Zusammensetzung einer Kristallstruktur vom Perowskit-Typ unter Verwendung eines Computers, wobei das computerimplementierte Entwurfsverfahren umfasst

   Bestimmen einer Kombination von $A_i$ und $B_i$ in der folgenden Formel (2), die durch Logarithmieren einer Formel eines Toleranzfaktors t erzeugt wird, dargestellt durch die folgende Formel (1), in der log t 0 ist, durch Ausführen einer Grundzustandssuche durch ein Temperverfahren unter Verwendung eines Ising-Modells oder QUBO,

$$t = \frac{(r_A + r_X)}{\sqrt{2}(r_B + r_X)} = \frac{d_{A-X}}{\sqrt{2}d_{B-X}} \text{ ... Formel (1)}$$

$$\log t = \sum_{i=1}^{n} A_i - \sum_{i=1}^{n} B_i \text{ ... Formel (2)}$$

wobei in Formel (1) $r_A$, $r_B$ und $r_X$ Ionenradien an einer A-Stelle, B-Stelle bzw. einer Anionenstelle darstellen, wenn eine allgemeine Formel der Kristallstruktur vom Perowskit-Typ durch ABX dargestellt wird, wobei A ein Kation darstellt, B ein Kation darstellt und X ein Anion darstellt, und $d_{A-X} = r_A + r_X$ und $d_{B-X} = r_B + r_X$ erfüllt sind, wobei in der Formel (2) $A_i$ und $B_i$ jeweils durch die folgenden Formeln (3) und (4) dargestellt werden,

$$\frac{1}{n} \log d_{A_i-X} \equiv A_i \text{ ... Formel (3)}$$

$$\frac{1}{n} \log \sqrt{2}d_{B_i-X} \equiv B_i \text{ ... Formel (4)}$$

wobei in den Formeln (2), (3) und (4) n der Parameter n in einer Zusammensetzungsformel $A^1_p A^2_q ... B^1_r B^2_x ... X_n$ ist, wobei p + q + ...= r + s + ... = n ist und jede Zahl ein ganze Zahl darstellt, und wobei sich die Ionen $A^1$, $A^2$, ... und $A^u$ des Zusammensetzungsverhältnisses p: q: ... an der A-Stelle befinden, und die Ionen $B^1$, $B^2$, ... und $B^v$ des Zusammensetzungsverhältnisses r: s : ... sich an der B-Stelle befinden in ABX, was die allgemeine Formel ist, und darüber hinaus die $d_{A-X}$ und $d_{Ai-X}$ in der Formel (3) die folgende Formel (5) erfüllen, und die $d_{B-X}$ und $d_{Bi-X}$ in die Formel (4) die folgende Formel (6) erfüllen,

$$d_{A-X} = \left(\prod_{i=1}^{n} \left(r_{A_i} + r_X\right)\right)^{\frac{1}{n}} = \left(\prod_{i=1}^{n} d_{A_i-X}\right)^{\frac{1}{n}} \text{ ... Formel (5)}$$

$$d_{B-X} = \left(\prod_{i=1}^{n} \left(r_{B_i} + r_X\right)\right)^{\frac{1}{n}} = \left(\prod_{i=1}^{n} d_{B_i-X}\right)^{\frac{1}{n}} \text{ ... Formel (6)}$$

wobei in den Formeln (5) und (6) n gleiche wie das n in den Formeln (2) bis (4) ist.

**5.** Computerimplementiertes Entwurfsverfahren nach Anspruch 4, wobei die Ionenart an der A-Stelle und die Ionenart an der B-Stelle auf eines oder mehrere spezifische Ionen eingestellt sind, und die Grundzustandssuche ausgeführt wird.

**6.** Computerimplementiertes Entwurfsverfahren nach Anspruch 4 oder 5, wobei ABX durch $ABO_{3-y}N_y$ dargestellt wird, wobei y eine ganze Zahl von 0 bis 3 darstellt.

## Revendications

**1.** Programme de conception pour amener un ordinateur à exécuter un processus de conception d'une composition d'une structure cristalline de type pérovskite, le programme de conception comprenant le fait d'amener un ordinateur à déterminer une combinaison de $A_i$ et $B_i$ dans la formule (2) suivante créée en prenant un logarithme d'une formule d'un facteur de tolérance, t, représenté par la suivante (1) suivante, dans laquelle logt vaut 0, en exécutant une recherche d'état fondamental par un procédé de recuit à l'aide d'un modèle d'Ising ou QUBO,

$$t = \frac{(r_A + r_X)}{\sqrt{2}(r_B + r_X)} = \frac{d_{A-X}}{\sqrt{2}d_{B-X}} \cdots \text{ Formule (1)}$$

$$\log t = \sum_{i=1}^{n} A_i - \sum_{i=1}^{n} B_i \cdots \text{Formule (2)}$$

dans lequel, dans la formule (1), $r_A$, $r_B$ et $r_X$ représentent des rayons ioniques au niveau du site A, du site B et d'un site anionique, respectivement, lorsqu'une formule générale de la structure cristalline de type pérovskite est représentée par ABX, dans laquelle A représente un cation, B représente un cation et X représente un anion, et $d_A$-x = $r_A$ + $r_X$ et $d_{B-X}$ = $r_B$ + $r_X$ sont satisfaits,
dans lequel, dans la formule (2), $A_i$ et $B_i$ sont représentés par les formules (3) et (4) suivantes, respectivement,

$$\frac{1}{n} \log d_{A_i-X} \equiv A_i \cdots \text{Formule (3)}$$

$$\frac{1}{n} \log \sqrt{2} d_{B_i-X} \equiv B_i \cdots \text{Formule (4)}$$

dans lequel, dans les formules (2), (3) et (4), n est le paramètre n dans une formule de composition $A^1_p A^2_q \cdots B^1_r B^2_s \cdots X_n$, où p + q + $\cdots$ = r + s + $\cdots$ = n et chaque nombre représente un nombre entier, et où les ions $A^1$, $A^2$, $\cdots$ et $A^u$ d'un rapport de composition p: q: $\cdots$ sont situés au niveau du site A, et les ions $B^1$, $B^2$, $\cdots$, et $B^v$ d'un rapport de composition r: s: $\cdots$ sont situés au niveau du site B dans l'ABX qui est la formule générale, et de plus, les $d_{A-X}$ et $d_{Ai-X}$ dans la formule (3) satisfont à la formule (5) suivante, et les $d_{B-X}$ et $d_{Bi-X}$ dans la formule (4) satisfont à la formule (6) suivante,

$$d_{A-X} = \left(\prod_{i=1}^{n} (r_{A_i} + r_X)\right)^{\frac{1}{n}} = \left(\prod_{i=1}^{n} d_{A_i-X}\right)^{\frac{1}{n}} \cdots \text{Formule (5)}$$

$$d_{B-X} = \left(\prod_{i=1}^{n} (r_{B_i} + r_X)\right)^{\frac{1}{n}} = \left(\prod_{i=1}^{n} d_{B_i-X}\right)^{\frac{1}{n}} \cdots \text{Formule (6)}$$

dans lequel, dans les formules (5) et (6), n est le même que n dans les formules (2) à (4).

2. Programme de conception selon la revendication 1, dans lequel le type d'ion au niveau du site A et le type d'ion au niveau du site B sont définis sur un ou plusieurs ions spécifiques, et la recherche d'état fondamental est exécutée.

3. Programme de conception selon la revendication 1 ou 2, dans lequel l'ABX est représenté par $ABO_{3-y}N_y$, dans lequel y représente un nombre entier de 0 à 3.

4. Procédé de conception mis en oeuvre par ordinateur pour concevoir une composition d'une structure cristalline de type pérovskite à l'aide d'un ordinateur, le procédé de conception mis en oeuvre par ordinateur comprenant

la détermination d'une combinaison de $A_i$ et $B_i$ dans la formule (2) suivante créée en prenant un logarithme d'une formule d'un facteur de tolérance, t, représenté par la formule (1) suivante, dans laquelle logt est 0, en exécutant une recherche d'état fondamental par un procédé de recuit à l'aide d'un modèle d'Ising ou QUBO,

$$t = \frac{(r_A + r_X)}{\sqrt{2}(r_B + r_X)} = \frac{d_{A-X}}{\sqrt{2} d_{B-X}} \cdots \text{Formule (1)}$$

$$\log t = \sum_{i=1}^{n} A_i - \sum_{i=1}^{n} B_i \cdots \text{Formule (2)}$$

dans lequel, dans la formule (1), $r_A$, $r_B$ et $r_X$ représentent des rayons ioniques au niveau du site A, du site B et d'un site anionique, respectivement, lorsqu'une formule générale de la structure cristalline de type pérovskite est représentée par ABX, dans laquelle A représente un cation, B représente un cation et X représente un anion, et $d_{A-X} = r_A + r_X$ et $d_{B-X} = r_B + r_X$ sont satisfaits,

dans lequel dans la formule (2), $A_i$ et $B_i$ sont représentés par les formules suivantes (3) et (4), respectivement,

$$\frac{1}{n} \log d_{A_i-X} \equiv A_i \; \cdots \; \text{Formule (3)}$$

$$\frac{1}{n} \log \sqrt{2} d_{B_i-X} \equiv B_i \; \cdots \; \text{Formule (4)}$$

dans lequel, dans les formules (2), (3) et (4), n est le paramètre n dans une formule de composition $A^1_p A^2_q \cdots B^1_r B^2_s \cdots X_n$, où $p + q + \cdots = r + s + \cdots = n$ et chaque nombre représente un nombre entier, et où les ions $A^1$, $A^2$, $\cdots$ et $A^u$ d'un rapport de composition p: q: $\cdots$ sont situés au niveau du site A, et les ions $B^1$, $B^2$, $\cdots$, et $B^v$ d'un rapport de composition r: s: $\cdots$ sont situés au niveau du site B dans l'ABX qui est la formule générale, et de plus, les $d_{A-X}$ et $d_{Ai-X}$ dans la formule (3) satisfont à la formule (5) suivante, et les $d_{B-X}$ et $d_{Bi-X}$ dans la formule (4) satisfont à la formule (6) suivante,

$$d_{A-X} = \left(\prod_{i=1}^{n}(r_{A_i} + r_X)\right)^{\frac{1}{n}} = \left(\prod_{i=1}^{n} d_{A_i-X}\right)^{\frac{1}{n}} \; \cdots \; \text{Formule (5)}$$

$$d_{B-X} = \left(\prod_{i=1}^{n}(r_{B_i} + r_X)\right)^{\frac{1}{n}} = \left(\prod_{i=1}^{n} d_{B_i-X}\right)^{\frac{1}{n}} \; \cdots \; \text{Formule (6)}$$

dans lequel, dans les formules (5) et (6), n est le même que n dans les formules (2) à (4).

5. Procédé de conception mis en oeuvre par ordinateur selon la revendication 4, dans lequel le type d'ion au niveau du site A et le type d'ion au niveau du site B sont définis sur un ou plusieurs ions spécifiques, et la recherche d'état fondamental est exécutée.

6. Procédé de conception mis en oeuvre par ordinateur selon la revendication 4 ou 5, dans lequel l'ABX est représenté par $ABO_{3-y}N_y$, dans lequel y représente un nombre entier de 0 à 3.

# FIG. 1

B SITE

ANION SITE

A SITE

## FIG. 2A

ANION SITE

A SITE

B SITE

## FIG. 2B

ANION SITE

A SITE

B SITE

# FIG. 3

S1 — CONVERSION OF FORMULA OF TOLERANCE FACTOR (t)

S2 — ANNEALING

# FIG. 4

100

STATE HOLDING UNIT — 111

STATE S

ENERGY CALCULATION UNIT — 112

CANDIDATE NUMBER [Ni]

ENERGY CHANGE VALUE {-ΔEi}

TRANSITION CONTROL UNIT — 114

TEMPERATURE VALUE T

TEMPERATURE CONTROL UNIT — 113

TRANSITION PROPRIETY f

TRANSITION NUMBER N

---

CANDIDATE NUMBER [Ni]

CANDIDATE GENERATION UNIT — 114a

CANDIDATE NUMBER [Ni]

ENERGY CHANGE VALUE {-ΔEi}

PROPRIETY DETERMINATION UNIT — 114b

TEMPERATURE VALUE T

RANDOM NUMBER GENERATION UNIT — 114d

CANDIDATE NUMBER [Ni]

TRANSITION PROPRIETY {fi}

TRANSITION DETERMINATION UNIT — 114c

CANDIDATE N

TRANSITION PROPRIETY f

EP 3 786 963 B1

# FIG. 5

# FIG. 6

SELECT ONE STATE TRANSITION AS CANDIDATE — S0001

DETERMINE PROPRIETY OF STATE TRANSITION BY COMPARING ENERGY CHANGE VALUE FOR STATE TRANSITION WITH PRODUCT OF TEMPERATURE VALUE AND RANDOM NUMBER VALUE — S0002

ADOPT STATE TRANSITION IF STATE TRANSITION IS PERMITTED, AND DO NOT ADOPT STATE TRANSITION IF STATE TRANSITION IS NOT PERMITTED — S0003

# FIG. 7

# FIG. 8

30

14
DISPLAY UNIT

12
MEMORY

15
INPUT UNIT

13
STORAGE UNIT
(HARD DISK)

16
OUTPUT UNIT

17
I/O INTERFACE
UNIT

19
NETWORK
INTERFACE UNIT

18

40

11
CPU

20
NETWORK
INTERFACE UNIT

# FIG. 9

**EP 3 786 963 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 110021371 A
- US 20120180453 A1

- US 2015060743 A1

**Non-patent literature cited in the description**

- **J. B. GOODENOUGH ; J. M. LONGO.** Landolt-Bornstein, Mew Series. Springer-Verlag, 1970, vol. 4, 126

- **R. D. SHANNON.** *Acta Crystallographica,* 1976, vol. A32, 751
- **Y. Q. JIA.** *Journal of Solid State Chemistry,* 1991, vol. 95, 184